(19) Europäisches Patentamt
European Patent Office
Office européen des brevets

(11) Veröffentlichungsnummer: **0 325 971 A2**

(12) **EUROPÄISCHE PATENTANMELDUNG**

(21) Anmeldenummer: **89100613.2**

(51) Int. Cl.4: **C12P 7/42 , C12P 41/00**

(22) Anmeldetag: **14.01.89**

(30) Priorität: **26.01.88 US 148470**

(43) Veröffentlichungstag der Anmeldung: **02.08.89 Patentblatt 89/31**

(84) Benannte Vertragsstaaten: **AT BE CH DE FR GB IT LI NL**

(71) Anmelder: **F. HOFFMANN-LA ROCHE & CO. Aktiengesellschaft**
**Postfach 3255**
**CH-4002 Basel(CH)**

(72) Erfinder: **Coffen, David Llewellyn**
**270 Ridgewood Avenue**
**Glenridge, N.J. 07028(US)**
Erfinder: **Kalaritis, Panayiotis**
**25 Evergreen Avenue**
**New Providence, N.J.07974(US)**
Erfinder: **Partridge, John Joseph**
**620 Airport Road**
**Chapel Hill No. Carolina 27514(US)**

(74) Vertreter: **Kellenberger, Marcus, Dr. et al**
**Grenzacherstrasse 124 Postfach 3255**
**CH-4002 Basel(CH)**

(54) **Verfahren zur Herstellung optisch reiner Hydroxyarylalkansäuren und -ester.**

(57) Enantiomer reine (2R)-2-Hydroxy-arylalkansäureester und (2S)-2-Hydroxy-arylalkansäuren der Formeln

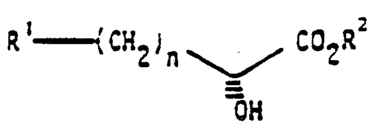
IA und
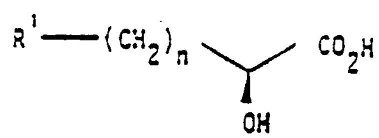
IB

worin $R^1$ Aryl, $R^2$ Alkyl, Aryl oder Aralkyl und n null oder eine Zahl von 1-8 bedeuten, werden durch Pseudomonas Lipase-katalysierte selektive Hydrolyse in Lösung oder in Suspension in einem wässrigen Medium bei einem kontrollierten pH-Wert zwischen ungefähr 5 und ungefähr 9 aus racemischen (2RS)-2-Hydroxy-arylalkansäureestern der Formel

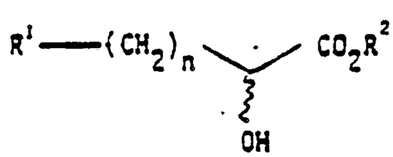
II
(R,S)

worin $R^1$, $R^2$ und n die oben angegebene Bedeutung besitzen, hergestellt.

EP 0 325 971 A2

## Verfahren zur Herstellung optisch reiner Hydroxyarylalkansäuren und -ester

Die vorliegende Erfindung betrifft ein neues Verfahren zur Herstellung optisch reiner (2R)-2-Hydroxy-arylalkansäureester und (2S)-2-Hydroxy-arylalkansäuren der allgemeinen Formeln

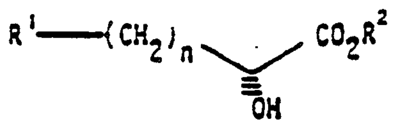

IA
(R)

und

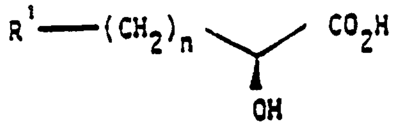

IB
(S)

worin $R^1$ Aryl, $R^2$ Alkyl, Aryl oder Aralkyl und n null oder eine Zahl von 1-8 bedeuten,
durch enzymatisch-kinetische Aufspaltung von racemischen (2RS)-2-Hydroxy-arylalkansäureestern der allgemeinen Formel

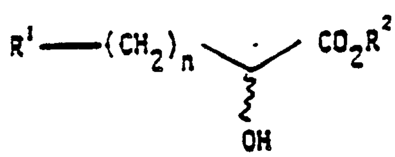

II

worin $R^1$, $R^2$ und n die oben angegebene Bedeutung besitzen.

Der in dieser Beschreibung verwendete Ausdruck "Alkyl" betrifft geradkettige und verzweigte Alkylgruppen, vorzugsweise Niederalkylgruppen mit 1-8 Kohlenstoffatomen, wie Methyl, Aethyl, Propyl, Isopropyl, Butyl und dergleichen.

Der in dieser Beschreibung ebenfalls verwendete Ausdruck "Aryl" betrifft monocyclische aromatische Kohlenwasserstoffgruppen, wie Phenyl, und polycyclische Arylgruppen, wie Naphthyl, Anthryl, Phenanthryl und dergleichen. Die bevorzugten Arylgruppen sind monocyclische Arylgruppen, insbesondere Phenyl.

Der Ausdruck "Aralkyl" betrifft geradkettige und verzweigte Alkylgruppen, vorzugsweise solche mit 1-8 Kohlenstoffatomen, welche in einer oben definierten Arylgruppe enden.

Jede der oben erwähnten Alkyl-, Aryl- oder Aralkylgruppen kann gegebenenfalls in einer oder mehreren Stellungen durch eine Reihe von Substituenten, wie Halogen, Alkoxy, Aryloxy, Thioalkoxy, Thioaryloxy und Alkyl, vorzugsweise Halogen (Chlor, Brom, Fluor oder Jod), substituiert sein.

In den in der vorliegenden Beschreibung angegebenen Formeln bedeutet eine dicke, spitzzulaufende Linie (▲) einen Substituenten mit $\beta$-Orientierung (d.h. oberhalb der Ebene des Moleküls bzw. der Seite), eine gestrichelte Linie (≡) einen Substituenten mit $\alpha$-Orientierung (d.h. unterhalb der Ebene des Moleküls bzw. der Seite) und eine Wellenlinie ( ≀ ) einen Substituenten mit $\alpha$- oder $\beta$-Orientierung oder Gemische dieser Isomeren.

Erfindungsgemäss wurde gefunden, dass das 2S-Enantiomere der Formel II selektiv zum 2S-Enantiomeren der Formel IB hydrolysiert wird, wenn man das racemische Gemisch der Formel II einer enzymatischen Hydrolyse unter Verwendung eines bakteriellen Lipase-Enzyms, welches aus einer Pseudomonas Spezies erhalten wurde, unterwirft. Die enzymtisch--kinetische Aufspaltung kann auch verwendet werden, um ein racemisches Gemisch der Formel II in das 2R-Enantiomere der Formal IA überzuführen.

Die erfindungsgemässe enzymtische Hydrolyse führt demnach zum 2R-Enantiomeren der Formel IA im Gemisch mit dem 2S-Enantiomeren der Formel IB. Diese Verbindungen können danach leicht nach üblichen Methoden getrennt werden.

Die Spezifität gewisser Mikroorganismen oder gewisser Enzyme, welche aus Mikroorganismen gewonnen wurden, ermöglicht deren potentielle Verwendung zur Herstellung von enantiomer reinen Zwischenprodukten aus racemischen Gemischen. Das erwünschte enantiomere Molekül kann danach in die Zielverbindung übergeführt werden. Die durch einen Mikroorganismus oder ein Enzym katalysierte Aufspaltung von Isomeren ergibt eine attraktive Alternative zu den mehr traditionellen und kostspieligen Methoden, wie chemische Aufspaltung und Hochdruck-Flüssigchromatographie diastereomerer Derivate.

Kato et al. haben berichtet, dass ein bekanntes Bakterium, nämliche Corynebacterium equi IFO 3730, die Eigenschaft besitzt, verschiedene Ester enantioselektiv zu hydrolysieren (Tetrahedron Letters., Band 28, Nr. 12, 1987, Seiten 1303-1306). In ihrer Studie wurde der Mikroorganismus für die asymmetrische Hydrolyse 2-Benzyloxy-substituierter Alkan- und Arylalkancarbonsäureester eingesetzt, wobei eine Suspension gewachsener Zellen von Corynebacterium equi und ein verlängerter (z.B. 24 Stunden) Fermentationsprozess zur Anwendung kamen. Die nicht reagierten Niederalkylester wurden in der optisch aktiven S-Form wiedergewonnen, und zwar in einer hohen enantiomeren Reinheit (über 99% e.R.). Es wurde auch festgestellt, dass der Austausch des Alkyl-oder Alkenylrests des Substrates mit einer Phenylmethylgruppe eine Umkehrung der Stereoselektivität verursachte, d.h. die Verbindung fiel in der optisch aktiven R-Form an, und zwar ebenfalls in hoher enantiomerer Reinheit.

Kitazume et al. haben ein Verfahren zur asymmetrischen Hydrolyse von 2-Fluor-2-methylmalonsäurediestern mit Esterase aus Schweineleber beschrieben, gemäss welchem die optisch aktiven (-)-2-Fluor-2-methylmalonsäuremonoester erhalten wurden, jedoch in niedriger enantiomerer Reinheit. Beschrieben wurde auch eine mikrobielle Hydrolyse von 2-Fluor-2-substituierten Malonsäurediestern mit Esterase und Cellulase, wobei die optisch aktiven (+)- oder (-)-2-Fluor-2-substituierten Malonsäuremonoester erhalten wurden (J. Org. Chem., 51, 1986, Seiten 1003-1006).

Gu et al. haben berichtet, dass optisch aktive 3-Benzoylthio-2-methylpropionsäuren durch mikrobielle Lipase-katalysierte enantioselektive Hydrolyse ihrer entsprechenden Ester hergestellt werden können. Enantioselektivität bezüglich des stereochemisch bevorzugten S-Isomeren war jedoch mit allen eingesetzten Lipasen schlecht, weshalb zum Erreichen einer höheren Stereoselektivität eine Aenderung in der Struktur des Aroylthio-Teils des Substrats notwendig war. Insbesondere konnten durch Einführung von Methoxygruppen in den Phenylring in den Stellungen 3 und 5 die Stereospezifität von Lipase, welche aus Mucor meihei erhalten wurde, verbessert werden (Tetrahedron Letters, Band 27, Nr. 43, 1986, Seiten 5203-5206).

Iuchijima et al. haben ein Verfahren zur Herstellung optisch aktiver 2-Chlor- und 2-Brom-substituierter Alkylester und Säuren durch asymmetrische Hydrolyse racemischer Gemische der Ester beschrieben, wobei die Mikroorganismen Rizopus, Mucor, Aspergillus, Candida, Pseudomonas, Alcaligenes, Achromobacter und Bacillus, oder aus diesen Mikroorganismen gewonnene Enzyme verwendet wurden (publizierte Japanische Patentanmeldung [Kokai] Nr. 57-94.295 [1982]).

Ebenfalls in der Literatur beschrieben wurde die Candida Lipase-katalysierte, enantioselektive Hydrolyse von racemischem 2-Chlorpropionsäureoctylester zur R-Form der 2-Chlorpropionsäure (Cambou und Klibanov, Appl. Biochem. Biotech., 9, 1984, Seite 255).

U.S. Patentschrift Nr. 4,668,628 (Dahod et al.) beschreibt ein Verfahren zur enzymatischen Spaltung racemischer Gemische von partiell wasserlöslichen Estern, welches darin besteht, dass man das racemische Gemisch mit einem Candida Lipase-Enzym in Kontakt bringt, um dieses enzymatisch zu hydrolysieren. Ein spezifisches Beispiel ist die Candia Lipase-katalysierte Hydrolyse von D,L-2-Chlorpropionsäuremethylester.

Ein Nachteil Lipase-katalysierter kinetischer Spaltungen ist insbesondere die Tatsache, dass die Spezifität des Enzyms für ein gegebenes Substrat oft nicht vorausgesagt werden kann, da keine nützlichen Modelle existieren, welche für eine Lipase-katalysierte kinetische Aufspaltung eines potentiellen Substrates eine Voraussage bezüglich der Stereochemie ermöglichen.

Bei der Durchführung der erfindungsgemässen enzymatischen Aufspaltung wird die Verbindung der Formel II in einem wässrigen Medium gelöst oder, falls notwendig, suspendiert. Wenn die Verbindung der Formel II in einem wässrigen Medium suspendiert wird, können auch noch Emulgiermittel verwendet werden, um die Emulsionsbildung zu verbessern oder zu erleichtern, wobei konventionelle Emulgiermittel für diesen Zweck verwendet werden können.

Die enzymatische Hydrolyse wird bei einem pH von ungefähr 5 bis ungefähr 9, vorzugsweise bei einem pH von ungefähr 6 bis ungefähr 8, durchgeführt. Um den pH-Wert des Reaktionsgemisches im vorgenannten Bereich halten zu können, kann jede übliche Methode verwendet werden. Unter den bevorzugten Methoden kann die Verwendung von Puffern oder die automatische Titration genannt werden.

Die enzymatische Hydrolyse wird bei einem pH von ungefähr 5 bis ungefähr 10, vorzugsweise bei einem pH von ungefähr 7 bis ungefähr 9, durchgeführt. Um den pH-Wert des Reaktionsgemisches im vorgenannten Bereich halten zu können, kann jede übliche Methode verwendet werden. Unter den bevorzugten Methoden kann die Verwendung von Puffern oder die automatische Titration genannt werden.

Bei der Durchführung dieser enzymatischen Hydrolyse wird das racemische Gemisch der Formel II in einem wässrigen Medium gelöst oder anderweitig dispergiert und mit bakteriellem Lipase-Enzym umgesetzt. Im allgemeinen ist es bevorzugt, das Enzym in einer katalytisch wirksamen Menge zu verwenden. Unbestrittenermassen wird die zum Erhalten bester Resultate notwendige Bestimmung einer katalytisch wirksamen Menge eines bestimmten Enzyms von Faktoren abhängen, welche einem Fachmann geläufig sind. Diese Faktoren umfassen die Menge Ausgangsmaterial, die Herkunft des Enzyms, die Aktivität des Enzyms, die Reinheit des Enzyms und dergleichen. Man kann zwar einen Ueberschuss einer katalytisch wirksamen Menge des bakteriellen Lipase-Enzyms verwenden, doch wird durch die Verwendung eines grossen Ueberschusses an Enzym keine Verbesserung des Resultats erreicht.

Wie oben erwähnt, liefert die enzymatische Hydrolyse der racemischen Verbindung der Formel II die Verbindung der Formel IA im Gemisch mit der Verbindung der Formel IB. Diese Verbindungen können leicht getrennt werden, wenn die enzymatische Hydrolyse einmal gestoppt ist, und zwar durch sofortige Extraktion des Reaktionsmediums mit einem geeigneten organischen Lösungsmittel. Jede übliche Methode zur Trennung kann verwendet werden, um die Verbindung der Formel IA von der Verbindung der Formel IB zu isolieren. Unter den üblichen Methoden zur Trennung dieser zwei Verbindungen können Extraktion und Destillation genannt werden.

Die nach dem erfindungsgemässen Verfahren erhältlichen Verbindungen sind nützliche Zwischenprodukte zur Herstellung von ACE-Hemmern (Angiotensine Converting Enzyme), die zur Behandlung von Bluthochdruck verwendet werden können. Verfahren zur Herstellung solcher ACE-Hemmer aus Verbindungen wie den erfindungsgemäss Herstellbaren sind dem Fachmann geläufig und sind in der Patentliteratur beschrieben, beispielsweise in der U.S. Patentschrift 4,474,694 (Oka et al.), U.S. Patentschrift 4,512,924 (Attwood et al.), U.S. Patentschrift 4,658,024 (Attwood et al.) und EPA 0 012 401 (Patchett at al.).

Die folgenden Beispiele illustrieren im weiteren die vorliegende Erfindung, ohne diese zu beschränken.

In diesen Beispielen basiert die enantiomere Reinheit (% e.R.) auf dem diastereomeren Verhältnis der Derivate von (2R)- und (2S)-2-Hydroxy-4-phenylbutansäureäthylester mit S-(+)-α-Methoxy-α-trifluormethylphenylacetylchlorid (Mosher's Reagens). Die (2R)- und (2S)-2-Hydroxy-4-phenylbutansäuren wurden verestert, indem man diese in eine katalytische Menge Schwefelsäure enthaltendem Aethanol vor der Derivatisierung während 2 Stunden zum Rückfluss erhitzt hat. Die Derivate mit Mosher's Reagens wurden hergestellt, indem man 2,4 mMol des Substrats und 5,2 mMol Mosher's Reagens in 2 ml Pyridin bei 0° C über Nacht gerührt hat. Das diastereomere Verhältnis wurde durch isotherme Gaschromatographie an einer OV-17 Kapillarkolonne bei 225° C bestimmt, vgl. J.A. Dale et al., J. Org. Chem. 36, 1969, Seite 2543.

## Beispiel 1

### Herstellung von racemischer 2-Hydroxy-4-phenylbutansäure

Ein 5 1-Dreihalskolben, ausgerüstet mit einem mechanischen Rührwerk, einem Thermometer und einem Wasserstoffadapter, wird mit 410,0 g roher racemischer 2-Hydroxy-4-phenyl-3-butansäure, 2,5 l Methanol und 3,4 g eines 1:1-Gemisches von 5% Palladium auf Kohle/Wasser-Dispersion beladen. Die Hydrierung erfolgt bei Normaldruck und 20-25° C und ist innerhalb 2 Stunden vollständig.

Das Reaktionsgemisch wird durch Hyflo-Diatomeenerde filtriert, und das Filtrat bei 45° C bei 9100 Pa eingedampft, wobei man 412,0 g rohe 2-Hydroxy-4-phenylbutansäure erhält. Das NMR-Spektrum dieser rohen Säure ist in Uebereinstimmung mit dessen theoretischer Struktur.

## Beispiel 2

### Herstellung von racemischem 2-Hydroxy-4-phenylbutansäureäthylester

Ein 5 1-Dreihalskolben, ausgerüstet mit einem mechanischen Rührwerk, einem Thermometer und einem Kühler, wird mit 412,0 g roher racemischer 2-Hydroxy-4-phenyl-3-butansäure, 3 l absolutem Aethanol und 10 ml konzentrierter Schwefelsäure beladen. Die Lösung wird 2 Stunden zum Rückfluss erhitzt und danach auf 23° C abgekühlt und über Nacht bei Raumtemperatur gerührt. Die Lösung wird danach

vorsichtig mit 50 g festem Natriumbicarbonat versetzt und 10 Minuten gerührt, um die Säure zu neutralisieren und den pH-Wert auf 7 einzustellen. Das erhaltene Reaktionsgemisch wird danach über 50 g wasserfreiem Kaliumcarbonat getrocknet und filtriert. Das Lösungsmittel wird bei 45°C und 9100 Pa abfiltriert, wobei man 470,0 g des rohen Esters erhält, welcher unter vermindertem Druck (195 Pa) bei 133-134°C destilliert wird, wobei man 310,0 g (65%) 2-Hydroxy-4-phenylbutansäureäthylester erhält.

## Beispiel 3

Enantioselektive Hydrolyse von racemischem 2-Hydroxy-4-phenylbutansäureäthylester mit Lipase-Enzym: Herstellung von (2R)-2-Hydroxy-4-phenylbutansäureäthylester und (2S)-2-Hydroxy-4-phenylbutansäure

### Methode A: 50% Hydrolyse

Ein 3 1-Dreihalskolen, ausgerüstet mit einem mechanischen Rührwerk, einer mit einem pH-Kontrollgerät verbundenen Elektrode und einer mit einer peristaltischen Pumpe verbundenen Zugabevorrichtung, wird mit 450 ml deionisiertem Wasser, 50 ml 0,05M wässrigem Phosphatpuffer (pH 7,0) und 52,0 g racemischem 2-Hydroxy-4-phenylbutansäureäthylester beladen. Das Gemisch wird einige Minuten gerührt um sicherzustellen, dass der pH-Wert unverändert konstant bei 7,0 bleibt. Dann werden 0,67 g (20'000 Einheiten) Pseudomonas Lipase-Enzym (P-30, Amano International Enzyme Co., Inc., Troy, Virginia) zugegeben, und bei pH 7,0 und Raumtemperatur unter Rühren hydrolysiert. Der pH-Wert wird konstant gehalten, indem man mit der peristaltischen Pumpe 1,0N wässrige Natriumlösung zugibt, wobei die Pumpe durch das pH-Kontrollgerät aktiviert wird. Die Reaktion wird bei einer 50%igen Umwandlung unterbrochen, wenn 125 ml 1,0N wässrige Natriumhydroxidlösung zugegeben wurden (nach 24 Stunden). Das Reaktionsgemisch wird dann mit dreimal 200 ml (total 600 ml) Diäthyläther extrahiert. Die vereinigten organischen Extrakte werden über wasserfreiem Natriumsulfat getrocknet, und das Lösungsmittel bei 45°C une 9100 Pa entfernt, wobei man 25,3 g (48,5%, 97% der Theorie) (2R)-2-Hydroxy-4-phenylbutansäureäthylester erhält, welcher eine enantiomere Reinheit von 91% aufweist, $[\alpha]_D^{25}$ = -7,8° (c 1,0, Aethanol). Die wässrige Phase wird mit 3N Salzsäure auf pH 2 angesäuert und mit dreimal 200 ml Diäthyläther extrahiert. Die vereinigten organischen Extrakte werden über wasserfreiem Natriumsulfat getrocknet, und das Lösungsmittel bei 45°C und 9100 Pa entfernt, wobei man 20,8 g (46%, 92% der Theorie) (2S)-2-Hydroxy-4-phenylbutansäure erhält, welche eine enantiomere Reinheit von 90,8% aufweist, $[\alpha]_D^{25}$ = +7,6° (c 1,0, Aethanol), Schmelzpunkt 109-110°C.

### Methode B: 55% Hydrolyse

Ein 3 1-Dreihalskolben, ausgerüstet mit einem mechanischen Rührwerk, einer mit einem pH-Kontrollgerät verbundenen Elektrode und einer mit einer peristaltischen Pumpe verbundenen Zugabevorrichtung, wird mit 450 ml deionisiertem Wasser, 50 ml 0,05M wässrigem Phosphatpuffer (pH 7,0) und 52,0 g racemischem 2-Hydroxy-4-phenylbutansäureäthylester beladen. Das Gemisch wird für einige Minuten gerührt um sicherzustellen, dass der pH-Wert unverändert konstant bei 7,0 bleibt. Dann wird 1,0 g (30'000 Einheiten) Pseudomonas Lipase-Enzym (P-30, Amano International Enzyme Co., Inc., Troy, Virginia) zugegeben, und bei pH 7,0 und Raumtemperatur unter Rühren hydrolysiert. Der pH-Wert wird durch Zugabe von 1,0N wässriger Natriumhydroxidlösung mittels der peristaltischen Pumpe, welche durch pH-Kontrollgerät aktiviert wird, konstant gehalten. Die Reaktion wird bei einer 55%igen Umwandlung unterbrochen, wenn 137.5 ml 1,0N wässrige Natriumhydroxidlösung zugegeben wurden (nach 25 Stunden). Das Reaktionsgemisch wird dann mit dreimal 200 ml (600 ml total) Diäthyläther extrahiert. Die vereinigten organischen Extrakte werden über wasserfreiem Natriumsulfat getrocknet, und das Lösungsmittel bei 45°C und 9100 Pa entfernt, wobei man 22,4 g (43%, 96% der Theorie) (2R)-2-Hydroxy-4-phenylbutansäureäthylester erhält, welcher eine enantiomere Reinheit von 99% aufweist, $[\alpha]_D^{25}$ = -8,4° (c 1,15, Aethanol). Die wässrige Phase wird mit 3N Salzsäure auf pH 2 angesäuert und mit dreimal 200 ml Diäthyläther extrahiert. Die vereinigten organischen Extrakte werden über wasser freiem Natriumsulfat getrocknet, und das Lösungsmittel bei 45°C und 9100 Pa entfernt, wobei man 22,7 g (50%, 91% der Theorie) (2S)-2-Hydroxy-4-phenylbutansäure erhält, welche eine enantiomere Reinheit von 73% aufweist, $[\alpha]_D^{25}$ = +5,7°, Schmelzpunkt 109-110°C.

Methode C: 35% Hydrolyse

Die Hydrolyse wird mit dem gleichen Hydroxy-substituierten Ester wie oben beschrieben, 0,34 g Pseudomonas Lipase-Enzym (P-30, Amano International Enzyme Co., Inc., Troy, Virginia), 50 ml 0,05M wässrigem Phosphatpuffer (pH 7,0) und 450 ml deionisiertem Wasser unter den gleichen Reaktionsbedingungen wie oben wiederholt. Die Reaktion wird bei einer 35%igen Umwandlung unterbrochen, wenn 85 ml 1,0N wässrige Natriumhydroxidlösung zugegeben wurden. Aufarbeiten in der üblichen Weise liefert die folgenden Resultate:

| | (2R)-2-Hydroxy-4-phenylbutansäureäthylester | (2S)-2-Hydroxy-4-phenylbutansäure |
|---|---|---|
| Gewicht (g) | 32,5 | 14,6 |
| % Ausbeute | 63 | 33 |
| % Theorie | 96 | 93 |
| $[\alpha]_D^{25}$ | -4,6° (c 1,5, Aethanol) | +7,8° (c 1,1, Aethanol) |
| (% e.R.) | 52 | 92 |

**Ansprüche**

1. Verfahren zur Herstellung eines 2R-Esters der allgemeinen Formel

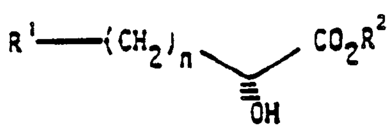

IA

und einer 2S-Carbonsäure der allgemeinen Formel

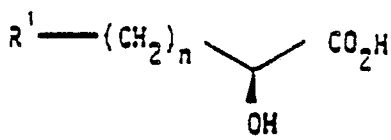

IB

worin $R^1$ Aryl, $R^2$ Alkyl, Aryl oder Aralkyl und n null oder eine Zahl von 1-8 bedeuten,
dadurch gekennzeichnet, dass man einen racemischen 2RS-Ester der allgemeinen Formel

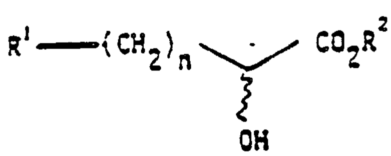

II

worin $R^1$, $R^2$ und n die oben angegebene Bedeutung besitzen,
mit einem bakteriellen Lipase-Enzym in einem wässrigen Medium zur selektiven Ueberführung des racemischen 2RS-Esters in den 2R-Ester und die 2S-Carbonsäure umsetzt, wobei die Umsetzung bei einem pH-Wert zwischen etwa 5 und etwa 9 durchgeführt wird, und danach den 2R-Ester und die 2S-Säure getrennt aus dem Reaktionsmedium isoliert.

2. Verfahren gemäss Anspruch 1, worin die Reaktion bei einem pH-Wert zwischen etwa 6 und etwa 8 durchgeführt wird.

3. Verfahren gemäss Anspruch 1 oder 2, worin $R^1$ Phenyl bedeutet.

4. Verfahren gemäss einem der Ansprüche 1-3, worin $R^2$ Niederalkyl bedeutet.

5. Verfahren gemäss Anspruch 4, worin $R^2$ Aethyl bedeutet.

6. Verfahren gemäss Anspruch 5, worin $R^1$ Phenyl, $R^2$ Aethyl und n die Zahl 2 bedeuten.

7. Verfahren gemäss einem der Ansprüche 1-6, worin das bakterielle Lipase-Enzym aus einer Pseudomonas Spezies gewonnen wurde.